(19) Europäisches Patentamt
European Patent Office
Office européen des brevets

(11) **EP 1 483 007 B1**

(12) **EUROPEAN PATENT SPECIFICATION**

(45) Date of publication and mention
of the grant of the patent:
**13.06.2012 Bulletin 2012/24**

(21) Application number: **03710150.8**

(22) Date of filing: **10.03.2003**

(51) Int Cl.:
**A61M 16/00** $^{(2006.01)}$

(86) International application number:
**PCT/IB2003/001403**

(87) International publication number:
**WO 2003/075990 (18.09.2003 Gazette 2003/38)**

(54) **A BREATHING APPARATUS WITH COMPUTATION OF THE AIRFLOW PROVIDED TO THE PATIENT USING ONLY PRESSURE SENSORS**

EIN BEATMUNGSGERÄT MIT BERECHNUNG DES DEM PATIENTEN ZUGEFÜHRTEN LUFTSTROMES NUR MIT HILFE VON DRUCKSENSOREN

APPAREIL D'AIDE RESPIRATOIRE DESTINE A CALCULER LE DEBIT D'AIR FOURNI UNIQUEMENT AU MOYEN DE CAPTEURS DE PRESSION

(84) Designated Contracting States:
**AT BE BG CH CY CZ DE DK EE ES FI FR GB GR HU IE IT LI LU MC NL PT RO SE SI SK TR**

(30) Priority: **08.03.2002 US 362441 P**

(43) Date of publication of application:
**08.12.2004 Bulletin 2004/50**

(73) Proprietor: **Kaerys S.A.**
**06300 Nice (FR)**

(72) Inventors:
• **DELACHE, Alain**
**F-06300 Nice (FR)**

• **DELACHE, Véronique**
**F-06300 Nice (FR)**

(74) Representative: **Damen, Daniel Martijn**
**Philips International B.V.**
**Intellectual Property & Standards**
**High Tech Campus 44**
**5656 AE Eindhoven (NL)**

(56) References cited:
**EP-A- 0 821 976      EP-A- 1 166 813**
**EP-A- 1 177 810      WO-A-92/11054**
**WO-A-98/57691      WO-A-02/053217**

**Description**

Technical field

This invention concerns the field of apparatus to assist a patient respiration and more specifically an apparatus able to calibrate the tubes connected to the patient's mask and to the blower and to determine the airflow of the patient's from pressure measurement and from the tube calibration.

Background art

In many treatments apparatus are used to provide patients with air. More frequently it is used for patients with a breathing deficiency caused for example by the weakness of the breathing system or by obstructive apneas during the sleep. In those cases it is important to control the pressure of the air delivered to the patient. With respiratory insufficient patients, apparatus providing air at a higher pressure help to compensate the weakness of the patients lungs. In the case of patients suffering of sleep apneas, providing the air at a higher pressure removes the obstruction of the upper airways.

In order to provide a correct treatment, it is required to accurately know the value of airflow the patient is provided with. Usually the apparatus determine the airflow by measuring airflow in the patient circuit (between the blower and the mask) using an airflow sensor.

Airflow sensors can be based on high sensitivity differential pressure sensors measuring the pressure drop across a low resistance or using pitot tubes, or hot wire airflow sensors. Another commonly used mean to evaluate the airflow is by measuring the blower parameters such as speed, current consumption and power.

EP1177810A discloses a device for ventilating a patient, the device comprising: a gas source providing pressurized gas, a gas distribution system, and a valve for regulating respiration of the gas.

Summary of the invention

The first object of the invention is to determine the airflow at the patient's mask, without using sensors like airflow sensors that are expensive or motor consumption or rotation sensors that are not accurate especially at low flows.

The invention thus concerns to assist a patient respiration by delivering air to a patient trough a mask, said mask being designed to be connected on one first extremity of a tube, said apparatus comprising :

- a control unit to adjust the pressure delivered by the blower of said apparatus,
- a first pressure sensor for sensing the pressure PM at the first tube extremity and being connected to the control unit, and
- a second pressure sensor for sensing the pressure PB at the air output of said blower and being connected to said control unit.

These elements are comprised in the apparatus in order that, when a tube is connected to the mask and connected to said apparatus on its second extremity, the air flowing from the apparatus to the mask, said control unit is able to calculate the airflow at said second extremity of the tube from the pressures PM and PB and from the airflow resistance coefficient $K_T$ of the tube

The apparatus according to the invention is able to calculate the coefficient $K_T$ by using a shell with a traversing hole having a known airflow resistance coefficient $K_s$. This enables to use tubes of different sizes, and even tubes with different standards of airflow resistance coefficients.

A further implementation of the apparatus according to the invention is that the pressure control unit comprises an estimation module connected to the means for detecting the patient's breathing parameters, in order that the estimation module is able to determine when the patient is inspiring or expiring and in response the pressure to apply to the patient's mask, so that the control unit adjust the pressure delivered by the blower.

Further implementations enable to modulate the pressure of the provided air in response to the patient's breathing parameters and events which occur in the patient's breathing.

Brief description of figures

The purposes, objects and characteristics of the invention will become more apparent from the following description when taken in conjunction with the accompanying drawings in which:

Figure 1 represents the apparatus according to the present invention when used according to a preferential implementation,

Figures 2 represents the flowchart of the calibration of a tube by the apparatus,

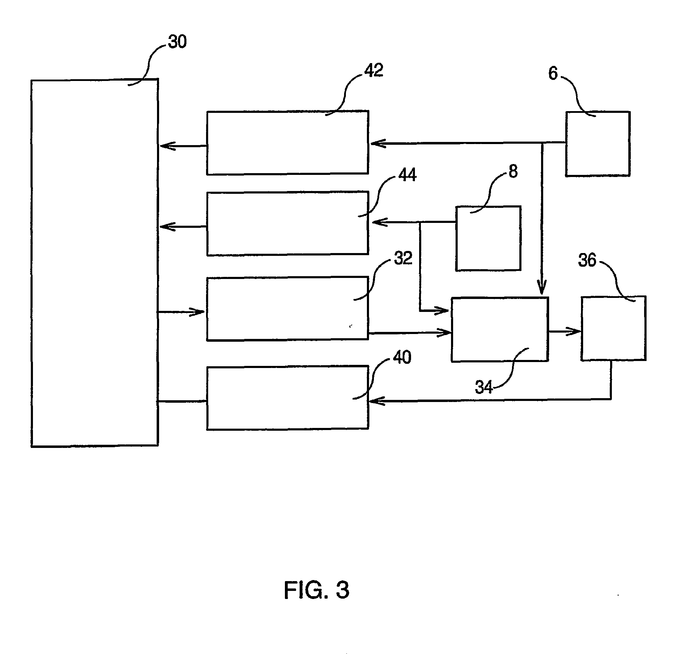

Figure 3 represents a device of the apparatus according to present invention,

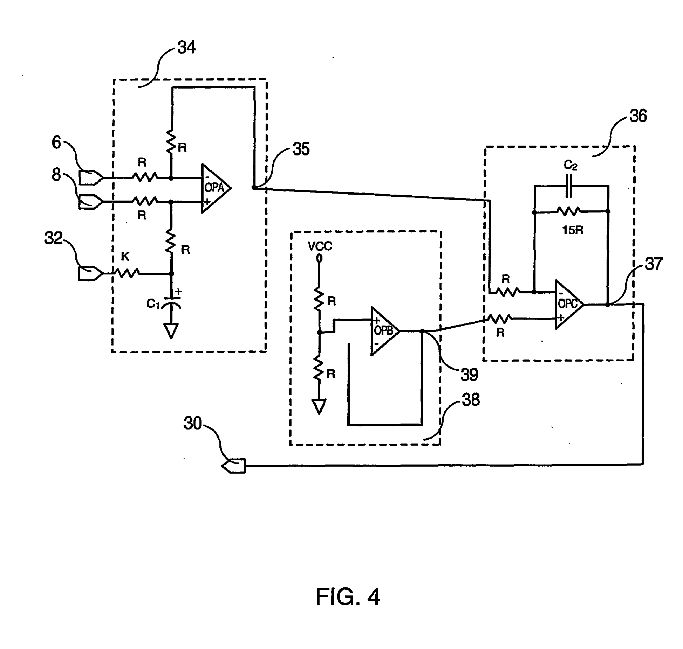

Figure 4 represents the electric schema of the device represented in figure 3,

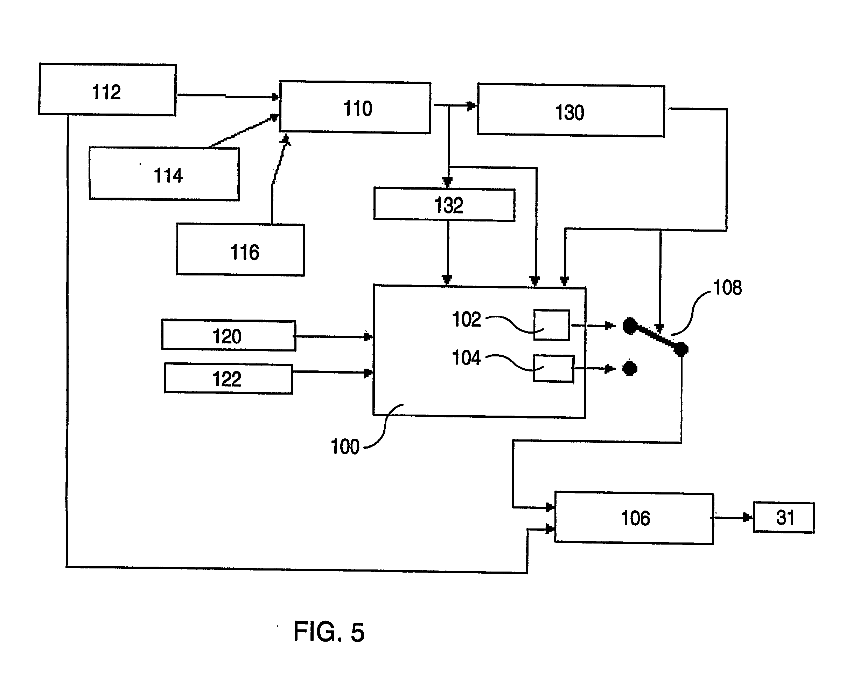

Figure 5 represents an implementation of the apparatus,

Figures 6 represents the way the apparatus reacts to events occurring in patient's breathing, and

Figure 7 represents how the apparatus operates to detect the presence of a patient at the mask.

Detailed description of the invention

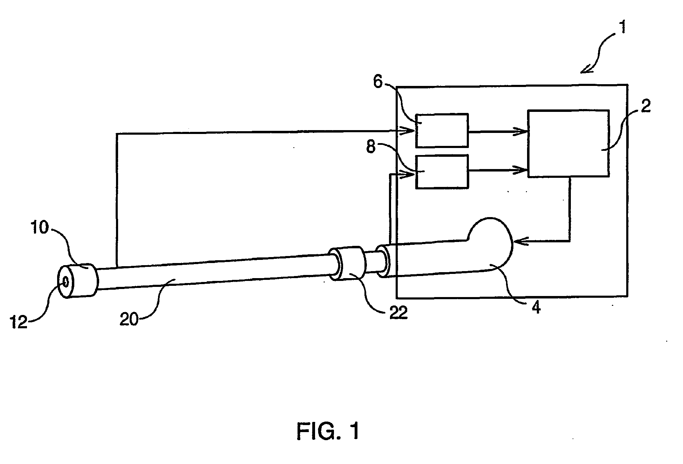

Ordinary tubes used in air assisting apparatus usually comprise a pressure sensing tube to measure the pressure at the end of the tube. The apparatus according to the present invention is based on this characteristic. As represented on figure 1, the apparatus 1 is connected to the tube 20 by the air inlet and connected to the pressure sensing tube of the tube. The pressure sensing tube is connected to a first pressure sensor 6 comprised in the apparatus. If no sensing tube is comprised, a mean will be connected at a first pressure sensor of the apparatus. Because the tube 20 diameter value is much smaller than the tube 20 length value, the pressure drop in the tube is defined by the following equation:

$$\Delta p = K_T \bullet airflow^2$$

wherein :

$K_T$ represents a constant coefficient characteristic of the tube,

$\Delta p$ represents the difference of pressure between the two tube extremities, and

airflow is the volume of air per time crossing the tube.

The apparatus has sensors that measure the pressure PB at the apparatus air outlet. As the apparatus is sensing pressure on both sides of the tube, knowing the tube coefficient k is allowing the system to compute the airflow by measuring the pressure drop. The present implementation consists in operating in a mode where precise airflow is required. A shell 10 which is a cap which comprises a small hole 12 at his top is placed to close one extremity of the tube, the other extremity of the tube being connected to the air outlet of the blower 4 of the apparatus 1. This calibration shell 10 has an airflow resistance coefficient $K_S$ which is a characteristic of it.

Considering that PB being the pressure sensed at the output of the apparatus and PM being the pressure sensed at the calibrated termination, S0 being known, we have :

$$Airflow = K_S \bullet \sqrt{PM}$$

as $\Delta p = K_T \bullet airflow^2$ then $K_T = \dfrac{(PB - PM)}{(K_S{}^2 \bullet PM)}$

The apparatus is thus able to determinate the tube coefficient $K_T$.

The calibration process takes advantages of having a number of measurements made at different pressures levels and by averaging them can get a more accurate $K_T$ value.

Example of a 1.8m tubing Ø15mm:

The calibration termination has a *S0* coefficient of **18** (airflow units are in LPM)

*PB* is measured at **9.90** hPa

*PM* is measured at **7.72** hPa

This is giving **k=0.000871**

Meaning then that the airflow is : **50.02** LPM

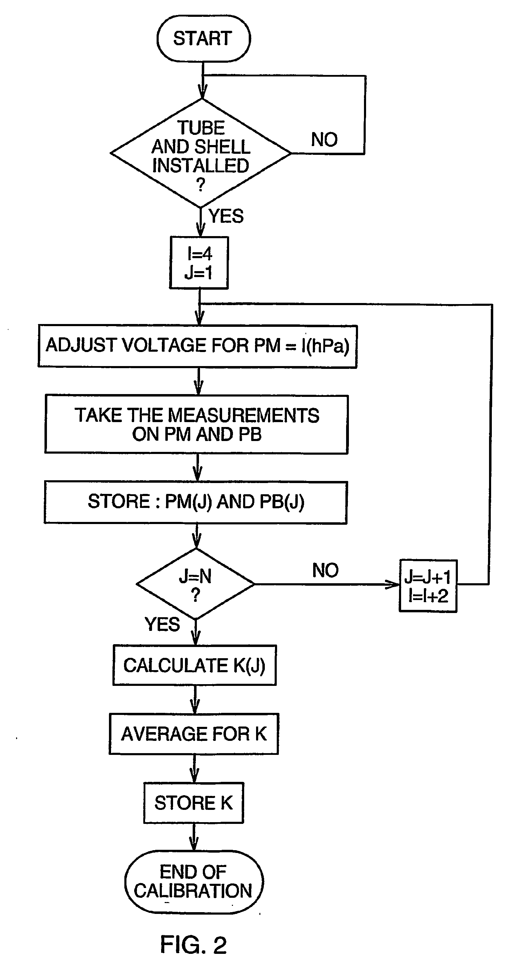

When using a new tube, the calibration process is entered, notably at the request of a clinician or a qualified user. The apparatus is expecting the calibration shell to be hooked as described in figure 1. The flowchart represented in figure 2 is showing an example of a series of measurements resulting on the $K_T$ coefficient calculation, then the tube coefficient $K_T$ is recorded and all the upcoming sessions will be based on this new tube coefficient. When the tube 20 and the shell 10 are installed, the value J corresponding to the number of one measure is set equal to 1 and associated to a number I corresponding to the value of the pressure provided by the blower 4, this I number being for example equal to 4 hPa when the calibration starts. Then the Pulse Width Modulation voltage is applied in order to deliver the pressure PM sensed at the calibrated termination which is the shell 10. The pressure PM and the pressure PB are measured and associated to the J number corresponding to the measure. If J does not equals the number of measures N required to calculate the $K_T$ average, the next measure is taken which means that J is incremented of 1. The next measure is preferentially made for a pressure incremented of 2 hPa, which means that I is incremented of two. When the number of measures required N is reached, the data associated to each measure are computed and enable to associate a tube coefficient $K_T$ value to each corresponding measure J. This enables to calculate an average of the $K_T$ value. For example if eight measures are required, J and I will be incremented 7 times before calculating the tube coefficient k value. This also enables to reject the tube if its standard does not correspond to the kind of tube which can be used with the apparatus 1. The $K_T$ value will then be used for the airflow computation.

The apparatus according to any of the previous claims, wherein the control unit comprises offset compensation means for compensating the possible difference of gauging between the two pressure

Another embodiment of the present invention is improving the sensitivity of the airflow measurement at low values, by gauging the pressure sensors.

Low flows accuracy is important in air delivery apparatus specially when triggering between inspiration and expiration where sensitivities as low as 5 l/mn are required.

A classical 22mm diameter breathing tube will present a pressure drop (PB-PM) of 0.01 hPa with an airflow of 5 l/mn, so it is mandatory to consider a signal amplification after the subtraction.

But this amplification cannot be done without a prior offset compensation. The apparatus according to the preferential implementation enables to correct this difference of gauging.

As previously described, using two pressure sensors at both ends of a breathing circuit tube, can give an accurate value of airflow., but due to physical constraints, for low flows accuracy, there is a need for amplification.

Due to the manufacturing process of airflow sensors, most of them are presenting a Voltage vs. Pressure relationship like:

$$Vout = Voffset + Kps \bullet \mathrm{Pr}$$

With

*Vout* being the output voltage

*Voffset* being the constant that can change drastically from one sensor to another within the same lot and that drifts slowly due to aging

*Kps* : the gain in volts/hPa of the pressure sensor that is usually stable.

*Pr* : the difference between pressure sensors 6 and 8

Given the previous equation, subtracting the two voltages of the two pressure sensors will then give:

$$VPbPm = VoffPm - VoffPb + Kps * (Pb - Pm)$$

With

**VPbPm** is the Voltage result of the subtraction

VoffPm is the offset of the Pm pressure sensor

VoffPb is the offset of the PB pressure sensor

Kps is the gain in volts/hPa of the pressure sensors

By offset it is mean the constant which corresponds to the difference between the pressure measured by one sensor and the absolute value of pressure.

The invention takes advantages of a well know state of the apparatus when no patient is connected to the apparatus, and no pressure is generated by the blower. During this state The PM and PB values have the same value than the ambient pressure. The control unit 2 comprises offset compensation means for compensating the possible difference of gauging between the two pressure sensors 6 and 8.

Preferentially the offset compensation means comprise :

- a digital to analog converter 32 connected to a microprocessor 30 in order to convert microprocessor's digital data in analog data,
- an analog substractor 34 having inputs connected to the second pressure sensor, to the first pressure sensor, and

4

to said digital to analog converter;

said microprocessor calculating, when the blower is not functioning, the difference between the two pressures measured by said first and second pressure sensors and then sending the value C of this difference to said digital to analog converter, which converts said value C in analog data and drive it to said analog subtractor, which subtract the pressure $P_B$ measured by said second pressure sensor and said value C to the pressure $P_M$ measured by said second pressure sensor and send the corresponding result D to the microprocessor, which will modify the C value until said D result equals zero, said microprocessor capturing the C value when said D result equals zero, enabling the microprocessor which uses the pressure sensors to control the apparatus to correct the difference of offsets between the pressure sensors.

Preferentially the apparatus comprises an analog amplifier 36 connected said analog subtractor 34 in order to amplify the signal corresponding to said D result and to send it to the microprocessor 30, thus enabling the microprocessor to have an accurate adjustment of said value C until the result D reaches the value zero.

The apparatus can also comprise analog to digital converters 42, 44 and 40 connected between the microprocessor 3) and the said first pressure sensor, between the microprocessor and the said second pressure sensor, and between the microprocessor and the said analog amplifier, so that the microprocessor is provided with only digital data.

With a non standard calibration tube, the process for calibrating a tube used in apparatus to assist patient's respiration, comprising :

- connecting a first tube's 20 extremity to the blower of an apparatus according to any of claim 1 to 5,
- if the tube is not provided with a pressure sensor at its second extremity, placing said second pressure sensor at said second extremity,
- connecting said second extremity to a shell 10 with a traversing hole 12 having a known airflow resistance coefficient $K_s$,
- switching the blower on, and instructing said control unit 2 to measured the pressures on both said first and second pressure sensors,
- calculating the value of the tube airflow resistance coefficient $K_t$ from these measured pressures and from the said coefficient $K_s$.

The apparatus can also comprise a Frequency Shift Keying (FSK) modulator which transforms the binary data send by the apparatus sensors or elements in a modulation of the frequency of the tension applied on a voltage controlled current source, connected to the external power supply, so that the voltage controlled current source transmit the modulation corresponding to the data, a FSK demodulator converting the voltage frequency modulation into binary data and transmit to the elements, so that each sensor or module connected to the power source is able to receive or transmit information.

The apparatus can also be used in a set for calibrating a tube used in apparatus to assist patient's respiration comprising the apparatus according to present invention and a shell (10) with a traversing hole (12) having a known airflow resistance coefficient $K_s$.

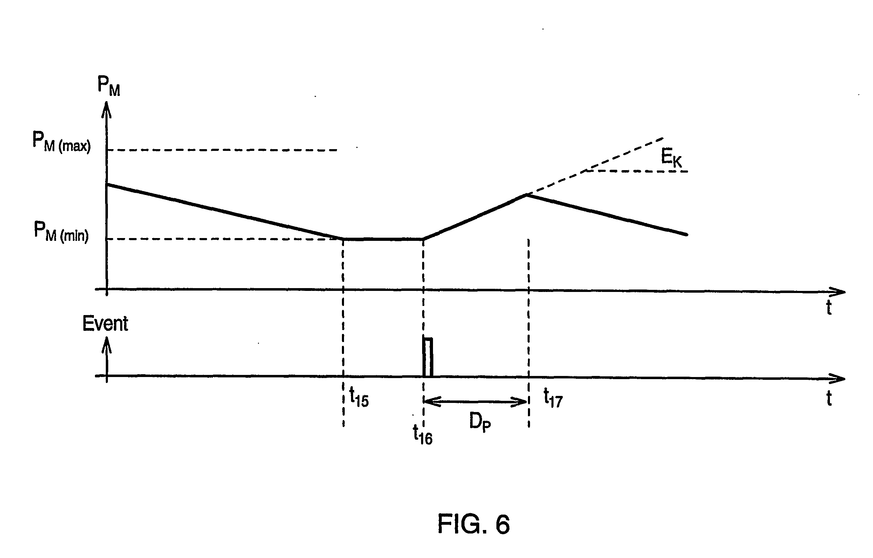

The apparatus enables to modulate the pressure to the patient in respect to the illness to treat. Due to the airflow computation, the apparatus has the capacity to differentiate the two basic states of the respiration: inspiration and expiration. The sensors provided in the apparatus enables the pressure control unit to control the pressure of the air delivered. The outputs of the E Estimator are the value of the inspiration pressure PI which is the pressure maintained at the patient's mask during the inspiration, and the value of the expiration pressure PE which is the pressure maintained at the patient's mask during the expiration. The data of the pressures PM and PB which are sensed at the extremities of the tube and the data of the tube coefficient $K_T$ enable the airflow computation. This computation enables the computation of the inspiration and expiration, this latest computation enables the estimation module 100 to determinate, which step of the patient's breathing is occurring. A breath estimation step is qualifying a breath in shape, energy (volume) and frequency. The clinician or a qualified user enters parameters of the delivered pressures for the expiration phase and the inspiration phase. The clinician enters also parameters defining how the estimation module 100 is going to react following events detected in the breath estimator. It is well known that a feedback of the patient with his treatment is helping compliance, thus the patient can have an access to a parameter ranging from min to max that is qualified to be "comfort vs. efficiency". This patient setting is having the weight that the clinician is giving to it, from pure placebo effect to some level of effects. Basically the patient settings are applied in the normal breath situation or/and have a limited action on the pressure regulation. It is also possible that the airflow is an input to the estimation module 100. Thus, with the data inputs concerning the breath estimation (and clinical symptoms or event associated with), the inspiration/expiration computation and the clinical settings, and possibly the airflow computation and patient settings, the control unit 2 by the estimation module 100 is able to determinate the pressures required PI and PE. Those two values can be addressed to two different outputs where a switch is able, relative to the inspiration/expiration computation, to connect to the required output regarding if the patient is breathing in or out. The control unit 2 comprises a pressure control loop which, by comparing the pressure measured in the mask and the value of pressure required PI or PE, is able to adjust the tension PWM in order to obtain the correct pressure in the mask. The figure 6 represents one pattern of the pressure of treatment provided according to the airflow due to the patient breathing. In this example, the clinical technician has set a special modulation of pressures Pi and Pe during respectively the inspiration and the expiration; after a while as no special event occurs the values of the two pressures are changed.

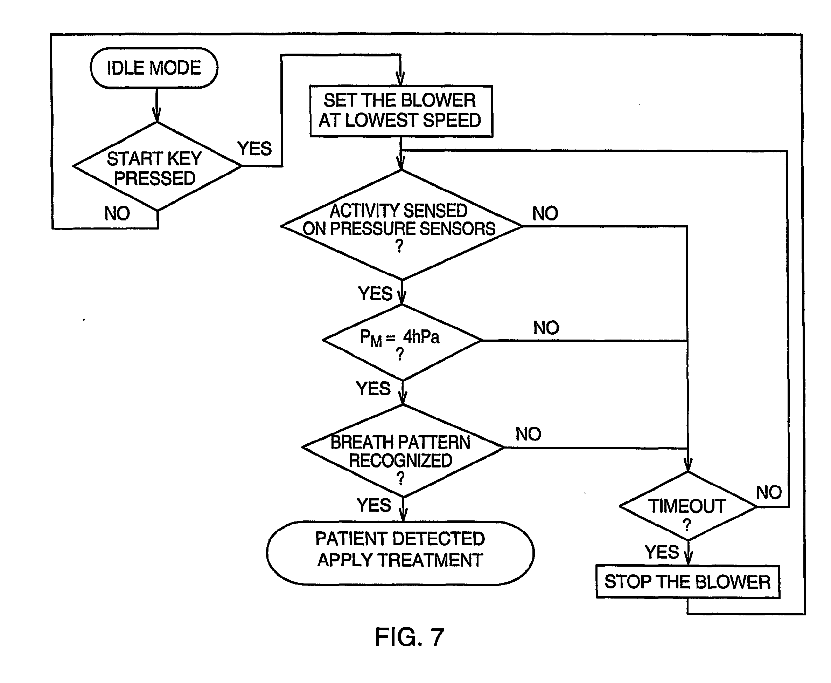

The apparatus has a two steps strong recognition process in order to prevent false start of the apparatus when the mask is not on the patient's face and to prevent starting a new treatment session. When the apparatus is started by the patient by using the keyboard, for example and as represented in figure 7 by using the start key, the blower 33 is kept turning at a very low speed, waiting for some activity on the mask pressure sensors 6. When an activity is detected, the apparatus is instantaneously trying to bring the pressure at the apparatus outlet at a minimum starting pressure SP of 4 hPa. When this pressure is reached the apparatus tries to identify at least one breath to start the process according to the settings. When the mask is not applied against something, like a hand or the patient's face, no activity is detected. Then if a maximum time since the apparatus start has been spend (the timeout is reached) then the blower is stopped. On the contrary, the apparatus keeps on waiting for an activity on the pressure sensors. When the .mask is not applied correctly the pressure can not reach 4 hPa. Then if the timeout is reached the blower is stopped, on the contrary the apparatus waits to detect some activity on the pressure sensors. When the mask is not applied on the patient's face no breath pattern is recognized. Then the blower is stopped if the timeout is reached. On the contrary the apparatus waits to detect some activity on the pressure sensors. The timeout checking prevents the blower keeping turning on if the patients does not start the treatment and forget to start the blower. A further advantage of this implementation is that if a patient is connected, bringing the pressure instantaneously to 4 hPa will prevent $CO_2$ rebreathing.

The following examples demonstrates the way the estimation module 100 modulates the pressure value $P_M$ to apply to the patient's mask.

Example 1 : Variations of the pressure of the average pressure of treatment as a function time according to detected events

In a preferential embodiment the average pressure of treatment on one breathing step is not constant in time and will be modulate by the estimation module 100 according to the events occurring, such as snoring or apneas.

The apparatus will try to reduce the average pressure of treatment value, thus enhancing the patient comfort while breathing against the apparatus. The clinician set a minimum average pressure of treatment Ptmin and a coefficient $N_{OEK}$ expressed in hPa/s.

As represented on figure 6 , when no events are detected the average pressure of treatment value will follow the equation:

$$PT(t) = MAX(PT(t-\varepsilon) - (NOEK \times \varepsilon), PT\min)$$

$\varepsilon$ being the sampling time, the MAX function is returning the greatest value of its two members.

The average pressure value PM, corresponding to the pressure of treatment PT on one breath thus decrease linearly until it reaches the minimum set by the clinician and stays constant until an event occurs. The average pressure is change each sampling time which correspond to one single breath (one inspiration and one consecutive expiration).

If an event is detected, a 3 steps process is initiated:

● Step 1: the estimation module, looking in the clinical settings is defining if the event has to affect the average value of pressure PT.
● Step 2 : If so the estimation module looking in clinical settings, will define a persistence delay $D_P$.
In the example of figure 6 a snore is detected, the persistence delay could be set to 2 minutes.
● Step 3 : A **Ek** parameter in hPa/s is extracted from clinical settings and balanced with an eventual ongoing event. The estimation module will determine the Ek corresponding to the event which occurs or has occurred and linearly increase the average pressure of treatment with Ek as slope coefficient. AVP will then follow the equation:

$$PT(t) = MIN(PT(t-\varepsilon) + (EK \times \varepsilon), AVP\max)$$

$\varepsilon$ being the sampling time, the MIN function is returning the smallest value of its two members.

During the persistence delay even if no event occurs the estimation module will keep on increasing the average pressure of treatment PT.

Example 2 : d1, d2 auto adjustment

On the figures 6 d1 and d2 have two different behaviors, according to t2 and t4 definitions. t2 is defined when the absolute value of airflow starts to decrease within the inspiration phase or shows a fixed delay after t1. t4 is defined when the absolute value of airflow starts to decrease within the expiration phase or shows a fixed delay after t3.

If t2 and t4 are defined according to the airflow waveform then no auto-adjustment is occurring. If not, following the same rules than AVP, the d1 and d2 can also be affected by the events. In the case when d1 & d2 are not locked by the breath waveform following the same process, d1 & d2 can also be adjusted according to events in the case.

**Claims**

1.    An apparatus (1) to assist a patient respiration by delivering air to a patient trough a mask, said mask being designed to be connected on one first extremity of a tube, said apparatus comprising :

    - a control unit (2) to adjust the pressure delivered by the blower (4) of said apparatus,
    - a first pressure sensor (6) for sensing the pressure PM at said first tube extremity and being connected to said control unit, and
    - a second pressure sensor (8) for sensing the pressure PB at the air output of said blower and being connected to said control unit;

    in order that, when a tube is connected to said mask and connected to said apparatus on its said second extremity, the air flowing from the apparatus to the mask, said control unit is able to calculate the airflow at said second extremity of the tube from said pressures PM and PB and from the airflow resistance coefficient $K_T$ of said tube ; wherein when a tube is connected between said apparatus (1) and a shell (10) with a traversing hole (12) having a known airflow resistance coefficient $K_s$, the air flowing from the apparatus to said shell, the measured pressures PM and PB are sent to said control unit (2) which calculates the tube airflow resistance coefficient $K_T$ from these measured pressures and from the said coefficient $K_s$.

2.    The apparatus according to claim 1, wherein the control unit (2) comprises offset compensation means for compensating the possible difference of gauging between the two pressure sensors (6 and 8).

3.    An apparatus (1) to assist a patient respiration by delivering air to a patient trough a mask, said mask being designed to be connected on one first extremity of a tube, said apparatus comprising :

    - a control unit (2) to adjust the pressure delivered by the blower (4) of said apparatus,
    - a first pressure sensor (6) for sensing the pressure PM at said first tube extremity and being connected to said control unit, and
    - a second pressure sensor (8) for sensing the pressure PB at the air output of said blower and being connected to said control unit;

    in order that, when a tube is connected to said mask and connected to said apparatus on its said second extremity, the air flowing from the apparatus to the mask, said control unit is able to calculate the airflow at said second extremity of the tube from said pressures PM and PB and from the airflow resistance coefficient $K_T$ of said tube ; wherein the control unit (2) comprises offset compensation means for compensating the possible difference of gauging between the two pressure sensors (6 and 8).

4.    The apparatus according to claim 3, wherein when a tube is connected between said apparatus (1) and a shell (10) with a traversing hole (12) having a known airflow resistance coefficient $K_s$, the air flowing from the apparatus to said shell, the measured pressures PM and PB are send to said control unit (2) which calculates the tube airflow resistance coefficient $K_T$ from these measured pressures and from the said coefficient $K_s$.

5.    An apparatus (1) according to claim 2 to 4, wherein said offset compensation means comprise :

    - a microprocessor (30)
    - a digital to analog converter (32) connected to said microprocessor (30) in order to convert microprocessor's digital data in analog data,
- an analog subtractor (34) having inputs connected to the second pressure sensor (8), to the first pressure sensor (6), and to said digital to analog converter,

said microprocessor calculating, when the blower is not functioning, the difference between the two pressures measured by said first and second pressure sensors and then sending the value C of this difference to said digital to analog converter, which converts said value C in analog data and drive it to said analog subtractor, which subtract the pressure PB measured by said second pressure sensor and said value C to the pressure PM measured by said second pressure sensor and send the corresponding result D to the microprocessor, which will modify the C value until said D result equals zero, said microprocessor capturing the C value when said D result equals zero, enabling the control unit to correct the difference of offsets between the pressure sensors.

6.  An apparatus according to claim 5, further comprising an analog amplifier (36) connected to said analog subtractor (34) in order to amplify the signal corresponding to said D result and to send it to said microprocessor (30), thus enabling said microprocessor to have an accurate adjustment of said value C until said result D reaches the value zero.

7.  An apparatus according to claim 6, further comprising analog to digital converters (42, 44 and 40) connected between the microprocessor (30) and the said first pressure sensor (6), between the microprocessor and the said second pressure sensor (8), and between the microprocessor and the said analog amplifier (36), so that the microprocessor is provided with only digital data.

8.  The apparatus according to any one of the precedent claims, wherein when at least one filter (22) is placed at one tube's extremity, said control unit (2) is able to calculate the airflow at said second extremity of the tube (20) from these measured pressures PM and PB and from the airflow resistance coefficient $K_T$ of said tube and from the airflow resistance coefficient $K_F$ of said filter.

9.  An apparatus according to any one of the precedent claims, wherein said control unit (2) comprises a non volatile memory in which the control unit stores, as a couple of values, the two pressures PM(J) and PB(J), measured at each said pressure sensors (6 and 8) when said control unit forces the blower to deliver a determined constant pressure I at one of the two sensors (6 or 8), so that when at least two couples of pressures corresponding to two different said determined constant pressure I are stored, the control unit is able to calculate an average of said coefficient $K_T$.

10. The apparatus according to any one of the precedent claims, wherein said control unit (2) comprises an estimation -module (100) connected to the means for detecting the patient's breathing parameters (110), in order that the estimation module is able to determine when the patient is inspiring or expiring and in response the pressure to apply to the patient's mask, so that the control unit adjust the pressure delivered by the blower.

11. The apparatus according to claim 10, wherein the control unit (2) comprises a non volatile memory (120) in which the clinician can enter clinical settings comprising at least the treatment pressure and possibly the pressure to apply according to the patient's breathing parameters, said estimation module (100) applying the pressure according to these clinical settings and to the patient's breathing parameters.

12. The apparatus according to claim 11, wherein the patient can enter patient settings (122) in said non volatile memory, said estimator applying the pressure according to these patient settings and to the patient's breathing parameters within bounds given by the clinician settings.

13. The apparatus according to any one of claim 10 to 12, in which the estimation module 100 is able to determine that an event ($E_1$, $E_2$ or $E_3$) occurs in patient's breathing thus enabling said control unit to adjust the tension to apply to the blower to adjust the pressure at patient's mask.

14. The apparatus according to any one of claim 10 to 13, wherein said means (6) for detecting the patient's breathing parameters enable the control unit (2) to compute the airflow at patient's mask (20), said comparator determining that an event ($E_1$, $E_2$ or $E_3$) is occurring with the airflow parameters or shape.

15. The apparatus according to claim 10 to 14, wherein said estimation module has an inspiration out put (102) where said estimation module set the mask pressure PM value during inspiration and wherein said estimation module has an expiration out put (102) where said estimation module set the mask pressure PM value during expiration, said control unit comprising a switch which is connected alternatively to the inspiration out put (102) or expiration out put (102) according to patient's breathing.

16. The apparatus according to claim 10 to 15, wherein the apparatus further comprise a starting mean which when actuated enables the estimation module (100) to determine if a breathing activity is detected, the estimator module sending the instruction to stop the blower if no activity is sensed after a given delay.

17. The apparatus according to any one of the previous claim, further comprising a Frequency Shift Keying (FSK) modulator 50 which transforms the binary data send by the apparatus sensors or elements in a modulation of the frequency of the tension applied on a voltage controlled current source 52, connected to the external power supply, so that the voltage controlled current source 52 transmit the modulation corresponding to the data, a FSK demodulator converting the voltage frequency modulation into binary data 61 and transmit to the elements, so that each sensor or module connected to the power source is able to receive or transmit information.

18. Set for calibrating a tube used in apparatus to assist patient's respiration comprising :

- an apparatus according to claim 1 to 6
- a shell (10) with a traversing hole (12) having a known airflow resistance coefficient $K_s$.

19. Process for calibrating a tube used in apparatus to assist patient's respiration by using the apparatus (1) according to any of claim 1 to 9, said process comprising :

- connecting a first tube's (20) extremity to the blower (4) of said apparatus,
- connecting said first pressure sensor (6) to measure the pressure PM at a second tube's extremity,
- connecting said second extremity to a shell (10). with a traversing hole (12) having a known airflow resistance coefficient $K_s$,
- switching the blower on,
- instructing said control unit (2) to measured the pressures on said first pressure sensor and on the second pressure sensor (8), which is measuring the pressure PB at the blower's apparatus outlet, and
- calculating the value of the tube airflow resistance coefficient $K_t$ from these measured pressures PM and PB and from the said coefficient $K_s$.

20. Process according to claim 19, further comprising the steps of:

- fixing, after the step of switching, at a value I the pressure provided and measured on one pressure sensor,
- storing, after the step of instructing, these measures PM(J) and PB(J) as a couple of measures associated to said value I,
- repeating a number of time N the steps of fixing and instructing of said process, said value I being different for each time, so that each couples of pressures is associated with one value I,

and wherein in the step of calculating an average of the airflow resistance coefficient $K_T$ is calculated from the measure pressures PM and PB and from the said coefficient $K_S$.

**Patentansprüche**

1. Vorrichtung (1) zur Unterstützung der Atmung eines Patienten durch die Zuführung von Luft zu einem Patienten durch eine Maske, wobei die genannte Maske so ausgelegt ist, dass sie an ein erstes Ende eines Schlauchs angeschlossen wird, wobei die genannte Vorrichtung Folgendes umfasst:

- ein Steuergerät (2) zum Anpassen des durch das Gebläse (4) der genannten Vorrichtung gelieferten Drucks,
- einen ersten Drucksensor (6), der mit dem genannten Steuergerät verbunden ist, zum Erfassen des Druckes PM am genannten ersten Schlauchende,
- einen zweiten Drucksensor (8), der mit dem genannten Steuergerät verbunden ist, zum Erfassen des Druckes PB am Luftaustritt des genannten Gebläses;

damit das genannte Steuergerät beim Anschluss eines Schlauchs an die genannte Maske und beim Anschluss seines genannten zweiten Endes an die genannte Vorrichtung, wobei die Luft von der Vorrichtung zu der Maske strömt, in der Lage ist, den Luftstrom an dem genannten zweiten Ende des Schlauchs aus den genannten Drücken PM und PB und aus dem Luftströmungs-Widerstandskoeffizient $K_T$ des genannten Schlauches zu berechnen; wobei die Messwerte der Drücke PM und PB beim Anschluss eines Schlauchs zwischen der genannten Vorrichtung (1) und einer Hülse (10) mit einer durchgehenden Bohrung (12) mit einem bekannten Luftströmungs-Widerstandskoeffizienten $K_S$, wobei die Luft von der Vorrichtung zu der genannten Hülse strömt, an das genannte Steuergerät (2) gesendet werden, das den Luftströmungs-Widerstandskoeffizienten $K_T$ des Schlauches aus diesen Druckmesswerten und aus dem genannten Koeffizienten $K_S$ berechnet.

2. Vorrichtung nach Anspruch 1, wobei das Steuergerät (2) Offsetausgleichsmittel zum Ausgleichen der möglichen Eichdifferenz zwischen den beiden Drucksensoren (6 und 8) umfasst.

3. Vorrichtung (1) zur Unterstützung der Atmung eines Patienten durch die Zuführung von Luft zu einem Patienten durch eine Maske, wobei die genannte Maske so ausgelegt ist, dass sie an ein erstes Ende eines Schlauchs angeschlossen wird, wobei die genannte Vorrichtung Folgendes umfasst:

   - ein Steuergerät (2) zum Anpassen des durch das Gebläse (4) der genannten Vorrichtung gelieferten Drucks,
   - einen ersten Drucksensor (6), der mit dem genannten Steuergerät verbunden ist, zum Erfassen des Druckes PM am genannten ersten Schlauchende,
   - einen zweiten Drucksensor (8), der mit dem genannten Steuergerät verbunden ist, zum Erfassen des Druckes PB am Luftaustritt des genannten Gebläses;

   damit das genannte Steuergerät beim Anschluss eines Schlauchs an die genannte Maske und beim Anschluss seines genannten zweiten Endes an die genannte Vorrichtung, wobei die Luft von der Vorrichtung zu der Maske strömt, in der Lage ist, den Luftstrom an dem genannten zweiten Ende des Schlauches aus den genannten Drücken PM und PB und aus dem Luftströmungs-Widerstandskoeffizient $K_T$ des genannten Schlauches zu berechnen; wobei das Steuergerät (2) Offsetausgleichsmittel zum Ausgleichen der möglichen Eichdifferenz zwischen den beiden Drucksensoren (6 und 8) umfasst.

4. Vorrichtung nach Anspruch 3, wobei die Messwerte der Drücke PM und PB beim Anschluss des Schlauchs zwischen der genannten Vorrichtung (1) und einer Hülse (10) mit einer durchgehenden Bohrung (12) mit einem bekannten Luftströmungs-Widerstandskoeffizienten $K_S$, wobei die Luft von der Vorrichtung zu der genannten Hülse strömt, an das genannte Steuergerät (2) gesendet werden, das den Luftströmungs-Widerstandskoeffizienten $K_T$ des Schlauches aus diesen Druckmesswerten und aus dem genannten Koeffizienten $K_S$ berechnet.

5. Vorrichtung (1) nach Anspruch 2 und 4, wobei die genannten Offsetausgleichsmittel Folgendes umfassen:

   - einen Mikroprozessor (30),
   - einen Digital-Analog-Wandler (32), der mit dem genannten Mikroprozessor (30) verbunden ist, um die digitalen Daten des Mikroprozessors in analoge Daten umzuwandeln,
   - einen analogen Subtrahierer (34), dessen Eingänge mit dem zweiten Drucksensor (8), dem ersten Drucksensor (6) und dem genannten Digital-Analog-Wandler verbunden sind,

   wobei der genannte Mikroprozessor bei ausgeschaltetem Gebläse die Differenz zwischen den von dem genannten ersten und dem genannten zweiten Drucksensor gemessenen beiden Drücken berechnet und dann den Wert C dieser Differenz an den genannten Digital-Analog-Wandler sendet, der den genannten Wert C in analoge Daten umwandelt und ihn dem genannten analogen Subtrahierer zuführt, der den von dem genannten zweiten Drucksensor gemessenen Druck PB und den genannten Wert C von dem von dem genannten ersten Drucksensor gemessenen Druck PM subtrahiert und das entsprechende Ergebnis D an den Mikroprozessor sendet, der den Wert C verändert, bis das genannte Ergebnis D gleich Null ist, wobei der genannte Mikroprozessor den Wert C erfasst, wenn das genannte Ergebnis D gleich Null ist, so dass das Steuergerät die Offsetdifferenz zwischen den Drucksensoren korrigieren kann.

6. Vorrichtung nach Anspruch 5, die ferner einen analogen Verstärker (36) umfasst, der mit dem genannten analogen Subtrahierer (34) verbunden ist, um das dem genannten Ergebnis D entsprechende Signal zu verstärken und es an den genannten Mikroprozessor (30) zu senden, so dass der genannte Mikroprozessor eine genaue Anpassung des genannten Wertes C haben kann, bis das genannte Ergebnis D den Wert Null erreicht.

7. Vorrichtung nach Anspruch 6, die ferner Analog-Digital-Wandler (42, 44 und 40) umfasst, die zwischen den Mikroprozessor (30) und den genannten ersten Drucksensor (6), zwischen den Mikroprozessor und den genannten zweiten Drucksensor (8) und zwischen den Mikroprozessor und den genannten analogen Verstärker (36) geschaltet sind, so dass dem Mikroprozessor nur mit digitalen Daten versorgt wird.

8. Vorrichtung nach einem der vorhergehenden Ansprüche, wobei das genannte Steuergerät (2), wenn mindestens ein Filter (22) an einem Ende des Schlauches platziert wird, in der Lage ist, den Luftstrom an dem genannten zweiten Ende des Schlauches (20) aus diesen Druckmesswerten PM und PB und aus dem Luftströmungs-Widerstandskoeffizienten $K_T$ des genannten Schlauchs und aus dem Luftströmungs-Widerstandskoeffizienten $K_F$ des genannten Filters zu berechnen.

9. Vorrichtung nach einem der vorhergehenden Ansprüche, wobei das genannte Steuergerät (2) einen nichtflüchtigen Speicher umfasst, in dem das Steuergerät als ein Wertepaar die beiden Drücke PM(J) und PB(J) speichert, die an jedem der genannten Drucksensoren (6 und 8) gemessen werden, wenn das genannte Steuergerät das Gebläse zwingt, einen vorbestimmten konstanten Druck I an einem der beiden Sensoren (6 oder 8) zu liefern, so dass in dem Fall, dass mindestens zwei Druckwertepaare, die zwei verschiedenen genannten bestimmten konstanten Druckwerten I entsprechen, gespeichert werden, das Steuergerät in der Lage ist, einen Mittelwert des genannten Koeffizienten $K_T$ zu berechnen.

10. Vorrichtung nach einem der vorhergehenden Ansprüche, wobei das genannte Steuergerät (2) ein Beurteilungsmoduls (100) umfasst, das mit den Mitteln zum Detektieren der Atmungsparameter (110) des Patienten verbunden ist, so dass das Beurteilungsmodul in der Lage ist zu ermitteln, wann der Patient einatmet oder ausatmet, und als Reaktion den Druck zu bestimmen, der auf die Maske des Patienten anzuwenden ist, so dass das Steuergerät den durch das Gebläse gelieferten Druck anpasst.

11. Vorrichtung nach Anspruch 10, wobei das Steuergerät (2) einen nichtflüchtigen Speicher (120) umfasst, in den der Arzt klinische Einstellungen eingeben kann, die zumindest den Behandlungsdruck und möglicherweise den entsprechend den Atmungsparametern des Patienten anzuwendenden Druck umfassen, wobei das genannte Beurteilungsmodul (100) den Druck entsprechend diesen klinischen Einstellungen und den Atmungsparametern des Patienten anwendet.

12. Vorrichtung nach Anspruch 11, wobei der Patient Patienteneinstellungen (122) in den genannten nichtflüchtigen Speicher eingeben kann, wobei das genannte Beurteilungsmodul den Druck entsprechend diesen Patienteneinstellungen und den Atmungsparametern des Patienten innerhalb der durch die klinischen Einstellungen vorgegebenen Grenzen anwendet.

13. Vorrichtung nach einem der Ansprüche 10 bis 12, wobei das Beurteilungsmodul (100) in der Lage zu ermitteln, dass ein Ereignis ($E_1$, $E_2$ oder $E_3$) in der Atmung des Patienten eintritt, so dass das genannte Steuergerät die dem Gebläse zuzuführende Spannung anpassen kann, um den Druck an der Maske des Patienten anzupassen.

14. Vorrichtung nach einem der Ansprüche 10 bis 13, wobei die genannten Mittel (6) zum Detektieren der Atmungsparameter des Patienten ermöglichen, dass das Steuergerät (2) den Luftstrom an der Maske (20) des Patienten berechnet, wobei der genannte Komparator anhand der Luftstromparameter oder der Form bestimmt, dass ein Ereignis ($E_1$, $E_2$ oder $E_3$) eintritt.

15. Vorrichtung nach Anspruch 10 bis 14, wobei das genannte Beurteilungsmodul einen Einatmungsausgang (102) aufweist, an dem das genannte Beurteilungsmodul den Wert PM für den Maskendruck während der Einatmung einstellt, und wobei das genannte Beurteilungsmodul einen Ausatmungsausgang (104) aufweist, an dem das genannte Schätzungsmodul den Wert PM für den Maskendruck während der Ausatmung einstellt, wobei das genannte Steuergerät einen Schalter umfasst, der entsprechend der Atmung des Patienten entweder mit den Einatmungsausgang (102) oder dem Ausatmungsausgang (104) verbunden ist.

16. Vorrichtung nach Anspruch 10 bis 15, wobei die Vorrichtung ferner Startmittel umfasst, die bei Betätigung bewirken, dass das Beurteilungsmodul (100) ermitteln kann, ob eine Atmungstätigkeit detektiert wird, wobei das Beurteilungsmodul den Befehl sendet, das Gebläse anzuhalten, wenn nach einer vorgegebenen Verzögerung keine Tätigkeit erfasst wird.

17. Vorrichtung nach einem der vorhergehenden Ansprüche, die ferner einen Frequenzumtastungsmodulator 50 umfasst, der die von den Sensoren oder Komponenten der Vorrichtung gesendeten binären Daten in eine Modulation der Frequenz der Spannung umwandelt, die an einer spannungsgesteuerten Stromquelle 52, die mit der externen Spannungsversorgung verbunden ist, anliegt, so dass die spannungsgesteuerte Stromquelle (52) die den Daten entsprechende Modulation überträgt, wobei ein Frequenzumtastungsdemodulator die Spannungsfrequenzmodulation in binäre Daten 61 umwandelt und an die Komponenten sendet, so dass jeder Sensor oder jedes Modul, die mit der Stromquelle verbunden sind, Informationen empfangen oder senden kann.

18. Anordnung zum Kalibrieren eines Schlauches, der in einer Vorrichtung zur Unterstützung der Atmung eines Patienten verwendet wird, die Folgendes umfasst:

- eine Vorrichtung nach Anspruch 1 bis 6,
- eine Hülse (10) mit einer durchgehenden Bohrung (12) mit einem bekannten Luftströmungs-Widerstandskoeffizienten $K_S$.

19. Verfahren zum Kalibrieren eines Schlauchs, der in einer Vorrichtung zur Unterstützung der Atmung eines Patienten verwendet wird, unter Verwendung der Vorrichtung (1) nach einem der Ansprüche 1 bis 9, wobei das genannte Verfahren Folgendes umfasst:

- Verbinden eines ersten Endes des Schlauchs (20) mit dem Gebläse (4) der genannten Vorrichtung,
- Verbinden des genannten ersten Drucksensors (6) zum Messen des Druckes PM mit dem zweiten Ende des Schlauches,
- Verbinden des genannten zweiten Endes mit einer Hülse (10) mit einer durchgehenden Bohrung (12) mit einem bekannten Luftströmungs-Widerstandskoeffizienten $K_S$,
- Einschalten des Gebläses,
- Instruieren des genannten Steuergerätes (2), die Drücke an dem genannten ersten Drucksensor und an dem zweiten Drucksensor (8) zu messen, der den Druck PB am Ausgang des Gebläses misst, und
- Berechnen des Wertes des Luftströmungs-Widerstandskoeffizienten $K_T$ des Schlauches aus diesen Druckmesswerten PM und PB und aus dem genannten Koeffizient $K_S$.

20. Verfahren nach Anspruch 19, das ferner die folgenden Schritte umfasst:

- nach dem Schritt des Einschaltens Festlegen des zugeführten und an einem Drucksensor gemessenen Druckes auf einen Wert I,
- nach dem Schritt des Instruierens Speichern dieser Messwerte PM(J) und PB(J) als ein Messwertepaar, dem der genannte Wert I zugeordnet ist,
- Wiederholen einer Anzahl von Malen N der Schritte des Festlegens und Instruierens des genannten Verfahrens, wobei der Wert I jedes Mal unterschiedlich ist, so dass jedes Druckwertepaar einem Wert I zugeordnet wird,

und wobei in dem Schritt des Berechnens ein Mittelwertes des Luftströmungs-Widerstandskoeffizienten $K_T$ aus den Druckmesswerten PM und PB und aus dem genannten Koeffizienten $K_S$ berechnet wird.

## Revendications

1. Appareil (1) pour aider la respiration d'un patient en distribuant de l'air à un patient à travers un masque, ledit masque étant conçu pour être raccordé au niveau d'une première extrémité d'un tube, ledit appareil comprenant :

- une unité de commande (2) pour régler la pression fournie par le ventilateur (4) dudit appareil,
- un premier capteur de pression (6) pour détecter la pression PM au niveau de ladite première extrémité de tube et connecté à ladite unité de commande, et
- un second capteur de pression (8) pour détecter la pression PB au niveau de la sortie d'air dudit ventilateur et connecté à ladite unité de commande ;

de sorte que, lorsqu'un tube est raccordé audit masque et raccordé audit appareil au niveau de sa dite seconde extrémité, l'air s'écoulant de l'appareil au masque, ladite unité de commande soit capable de calculer l'écoulement d'air au niveau de ladite seconde extrémité du tube à partir desdites pressions PM et PB et à partir du coefficient de résistance à l'écoulement d'air $K_T$ dudit tube ;
dans lequel, lorsqu'un tube est raccordé entre ledit appareil (1) et une enveloppe (10) avec un trou traversant (12) possédant un coefficient de résistance à l'écoulement d'air connu $K_S$, l'air s'écoulant de l'appareil à ladite enveloppe, les pressions mesurées PM et PB sont envoyées à ladite unité de commande (2) qui calcule le coefficient de résistance à l'écoulement d'air $K_T$ du tube à partir de ces pressions mesurées et à partir dudit coefficient $K_s$.

2. Appareil selon la revendication 1, dans lequel l'unité de commande (2) comprend des moyens de compensation de décalage pour compenser l'éventuelle différence de calibrage entre les deux capteurs de pression (6 et 8).

3. Appareil (1) pour aider la respiration d'un patient en distribuant de l'air à un patient à travers un masque, ledit masque étant conçu pour être raccordé au niveau d'une première extrémité d'un tube, ledit appareil comprenant :

    - une unité de commande (2) pour régler la pression fournie par le ventilateur (4) dudit appareil,
    - un premier capteur de pression (6) pour détecter la pression PM au niveau de ladite première extrémité de tube et connecté à ladite unité de commande, et
    - un second capteur de pression (8) pour détecter la pression PB au niveau de la sortie d'air dudit ventilateur et connecté à ladite unité de commande ;

de sorte que, lorsqu'un tube est raccordé audit masque et raccordé audit appareil au niveau de sa dite seconde extrémité, l'air s'écoulant de l'appareil au masque, ladite unité de commande soit capable de calculer l'écoulement d'air au niveau de ladite seconde extrémité du tube à partir desdites pressions PM et PB et à partir du coefficient de résistance à l'écoulement d'air $K_T$ dudit tube ;
dans lequel l'unité de commande (2) comprend des moyens de compensation de décalage pour compenser l'éventuelle différence de calibrage entre les deux capteurs de pression (6 et 8).

4. Appareil selon la revendication 3, dans lequel, lorsqu'un tube est raccordé entre ledit appareil (1) et une enveloppe (10) avec un trou traversant (12) possédant un coefficient de résistance à l'écoulement d'air connu $K_s$, l'air s'écoulant de l'appareil à ladite enveloppe, les pressions mesurées PM et PB sont envoyées à ladite unité de commande (2) qui calcule le coefficient de résistance à l'écoulement d'air $K_T$ du tube à partir de ces pressions mesurées et à partir dudit coefficient $K_s$.

5. Appareil (1) selon la revendication 2 à 4, dans lequel lesdits moyens de compensation de décalage comprennent :

    - un microprocesseur (30),
    - un convertisseur numérique-analogique (32) connecté audit microprocesseur (30) afin de convertir des données numériques du microprocesseur en données analogiques,
    - un soustracteur analogique (34) possédant des entrées connectées au second capteur de pression (8), au premier capteur de pression (6), et audit convertisseur numérique-analogique,

ledit microprocesseur calculant, lorsque le ventilateur ne fonctionne pas, la différence entre les deux pressions mesurées par lesdits premier et second capteurs de pression et puis envoyant la valeur C de cette différence audit convertisseur numérique-analogique, qui convertit ladite valeur C en données analogiques et l'envoie audit soustracteur analogique, qui soustrait la pression PB mesurée par ledit second capteur de pression et ladite valeur C à la pression PM mesurée par ledit second capteur de pression et envoie le résultat correspondant D au microprocesseur, qui modifiera la valeur C jusqu'à ce que ledit résultat D soit égal à zéro, ledit microprocesseur capturant la valeur C lorsque ledit résultat D est égal à zéro, permettant à l'unité de commande de corriger la différence de décalages entre les capteurs de pression.

6. Appareil selon la revendication 5, comprenant en outre un amplificateur analogique (36) connecté audit soustracteur analogique (34) afin d'amplifier le signal correspondant audit résultat D et de l'envoyer audit microprocesseur (30), permettant ainsi audit microprocesseur de posséder un réglage précis de ladite valeur C jusqu'à ce que ledit résultat D atteigne la valeur zéro.

7. Appareil selon la revendication 6, comprenant en outre des convertisseurs analogiques-numériques (42, 44 et 40) connectés entre le microprocesseur (30) et ledit premier capteur de pression (6), entre le microprocesseur et ledit second capteur de pression (8), et entre le microprocesseur et ledit amplificateur analogique (36), de sorte que seulement des données numériques soient fournies au microprocesseur.

8. Appareil selon une quelconque des revendications précédentes, dans lequel lorsqu'au moins un filtre (22) est positionné au niveau de l'extrémité d'un tube, ladite unité de commande (2) est capable de calculer l'écoulement d'air au niveau de ladite seconde extrémité du tube (20) à partir de ces pressions mesurées PM et PB et à partir du coefficient de résistance à l'écoulement d'air $K_T$ dudit tube et à partir du coefficient de résistance à l'écoulement d'air $K_F$ dudit filtre.

9. Appareil selon une quelconque des revendications précédentes, dans lequel ladite unité de commande (2) comprend une mémoire non volatile dans laquelle l'unité de commande stocke, sous forme de paire de valeurs, les deux pressions PM(J) et PB(J), mesurées au niveau de chaque dit capteur de pression (6 et 8) lorsque ladite unité de commande force le ventilateur à fournir une pression constante déterminée I au niveau d'un des deux capteurs (6 ou 8), de sorte que, lorsqu'au moins deux paires de pressions correspondant à deux dites pressions constantes déterminées différentes I sont stockées, l'unité de commande soit capable de calculer une moyenne dudit coefficient $K_T$.

10. Appareil selon une quelconque des revendications précédentes, dans lequel ladite unité de commande (2) comprend un module d'estimation (100) connecté aux moyens pour détecter les paramètres de respiration du patient (110), de sorte que le module d'estimation soit capable de déterminer l'instant auquel le patient aspire ou expire et, en réponse, la pression à appliquer sur le masque du patient, de sorte que l'unité de commande règle la pression fournie par le ventilateur.

11. Appareil selon la revendication 10, dans lequel l'unité de commande (2) comprend une mémoire non volatile (120) dans laquelle le clinicien peut entrer des réglages cliniques comprenant au moins la pression de traitement et éventuellement la pression à appliquer selon les paramètres de respiration du patient, ledit module d'estimation (100) appliquant la pression selon ces réglages cliniques et selon les paramètres de respiration du patient.

12. Appareil selon la revendication 11, dans lequel le patient peut entrer des réglages de patient (122) dans ladite mémoire non volatile, ledit estimateur appliquant la pression selon ces réglages de patient et selon les paramètres de respiration du patient au sein de limites fournies par les réglages de clinicien.

13. Appareil selon une quelconque des revendications 10 à 12, dans lequel le module d'estimation (100) est capable de déterminer qu'un événement ($E_1$, $E_2$ ou $E_3$) se produit dans la respiration du patient, permettant ainsi à ladite unité de commande de régler la tension à appliquer sur le ventilateur pour régler la pression au niveau du masque du patient.

14. Appareil selon une quelconque des revendications 10 à 13, dans lequel lesdits moyens (6) pour détecter les paramètres de respiration du patient permettent à l'unité de commande (2) de calculer l'écoulement d'air au niveau du masque du patient (20), ledit comparateur déterminant qu'un événement ($E_1$, $E_2$ ou $E_3$) se produit avec les paramètres ou la forme d'écoulement d'air.

15. Appareil selon les revendications 10 à 14, dans lequel ledit module d'estimation comporte une sortie d'aspiration (102) où ledit module d'estimation règle la valeur de la pression de masque PM au cours de l'aspiration et dans lequel ledit module d'estimation comporte une sortie d'expiration (104) où ledit module d'estimation règle la valeur de la pression de masque PM au cours de l'expiration, ladite unité de commande comprenant un commutateur qui est connecté en alternance à la sortie d'aspiration (102) ou à la sortie d'expiration (104) selon la respiration du patient.

16. Appareil selon les revendications 10 à 15, dans lequel l'appareil comprend en outre un moyen de démarrage qui, lorsqu'il est mis en marche, permet au module d'estimation (100) de déterminer si une activité de respiration est détectée, le module estimateur envoyant l'instruction pour arrêter le ventilateur si aucune activité n'est détectée après un délai donné.

17. Appareil selon une quelconque des revendications précédentes, comprenant en outre un modulateur par déplacement de fréquence (« Frequency Shift Keying » ou FSK) (50) qui transforme les données binaires envoyées par les capteurs ou éléments de l'appareil en une modulation de la fréquence de la tension appliquée sur une source de courant à tension commandée (52), connectée à l'alimentation électrique externe, de sorte que la source de courant à tension commandée (52) transmette la modulation correspondant aux données, un démodulateur FSK convertissant la modulation de fréquence de tension en données binaires (61), et réalise une transmission aux éléments, de sorte que chaque capteur ou module connecté à la source d'alimentation électrique puisse recevoir ou transmette des informations.

18. Ensemble pour calibrer un tube utilisé dans un appareil pour aider la respiration d'un patient, comprenant :

un appareil selon les revendications 1 à 6,
une enveloppe (10) avec un trou traversant (12) possédant un coefficient de résistance à l'écoulement d'air connu $K_s$.

19. Procédé pour calibrer un tube utilisé dans un appareil pour aider la respiration d'un patient en utilisant l'appareil (1) selon une quelconque des revendications 1 à 9, ledit procédé comprenant :

- le raccord de l'extrémité d'un premier tube (20) au ventilateur (4) dudit appareil,
- la connexion dudit premier capteur de pression (6) pour mesurer la pression PM au niveau d'une extrémité d'un second tube,
- le raccord de ladite seconde extrémité à une enveloppe (10) avec un trou traversant (12) possédant un coefficient de résistance à l'écoulement d'air connu $K_s$,
- l'allumage du ventilateur,
- l'ordre, à ladite unité de commande (2), de mesurer les pressions sur ledit premier capteur de pression et sur le second capteur de pression (8), qui mesure la pression PB au niveau de la sortie d'appareil du ventilateur, et
- le calcul de la valeur du coefficient de résistance à l'écoulement d'air $K_t$ du tube à partir de ces pressions mesurées PM et PB et à partir dudit coefficient $K_s$.

20. Procédé selon la revendication 19, comprenant en outre les étapes suivantes :

- la fixation, après l'étape d'allumage, à une valeur I de la pression fournie et mesurée sur un capteur de pression,
- le stockage, après l'étape d'instruction, de ces mesures PM(J) et PB(J) sous forme de paire de mesures associées à ladite valeur I,
- la répétition un nombre de fois N des étapes de fixation et d'ordre dudit procédé, ladite valeur I étant différente à chaque fois, de sorte que chaque paire de pressions soit associée à une valeur I,

et dans lequel, dans l'étape de calcul, une moyenne du coefficient de résistance à l'écoulement d'air $K_T$ est calculée à partir des pressions mesurées PM et PB et à partir dudit coefficient $K_s$.

FIG. 1

FIG. 2

FIG. 3

FIG. 4

FIG. 5

FIG. 6

EP 1 483 007 B1

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1177810 A